# EUROPÄISCHE PATENTSCHRIFT

(11) **EP 3 463 566 B1**
(45) Veröffentlichungstag und Bekanntmachung des Hinweises auf die Patenterteilung: **07.10.2020**
(21) Anmeldenummer: 17726626.9
(22) Anmeldetag: 30.05.2017
(51) Int. Cl.: A61N 1/375, A61N 1/05, A61N 1/36

(54) **FORMADAPTIVES MEDIZINISCHES IMPLANTAT**
SHAPE-ADAPTIVE MEDICAL IMPLANT
IMPLANT MÉDICAL DE FORME ADAPTABLE

(30) Priorität: 01.06.2016 DE 102016110137
(43) Veröffentlichungstag der Anmeldung: 10.04.2019
(73) Patentinhaber: Medizinische Hochschule Hannover, 30625 Hannover (DE)
(72) Erfinder: STIEGHORST, Jan, 29352 Adelheidsdorf (DE); DOLL, Theodor, 30916 Isernhagen (DE)
(74) Vertreter: Gramm, Lins & Partner Patent- und Rechtsanwälte PartGmbB
(86) Internationale Anmeldenummer: PCT/EP2017/063024
(87) Internationale Veröffentlichungsnummer: WO 2017/207561

(56) Entgegenhaltungen:
- WO-A1-2015/030734
- WO-A1-2015/188805
- US-A1- 2006 058 861
- US-A1- 2008 058 914
- US-A1- 2011 106 101
- US-A1- 2011 319 907
- US-B2- 8 145 326
- US-B2- 8 843 216

## Beschreibung

Die Erfindung betrifft ein formadaptives medizinisches Implantat mit wenigstens einem Aktor, durch den das Implantat von einer ersten Implantat-Geometrie in eine zweite Implantat-Geometrie verstellt werden kann, wobei das Implantat in der zweiten Implantat-Geometrie eine andere geometrische Form aufweist als in der ersten Implantat-Geometrie, wobei der Aktor eine aufquellbare chemische Substanz aufweist, die in Folge von Flüssigkeitszufuhr aufquillt, und wobei das Implantat wenigstens ein Flüssigkeitstransportmittel aufweist, das zur Zuführung von außerhalb des Implantats vorhandener Flüssigkeit zu der aufquellbaren chemischen Substanz eingerichtet ist. Die Erfindung betrifft außerdem die Verwendung einer elektrischen Signalquelle gemäß Anspruch 10. Die Erfindung betrifft somit das Gebiet der medizinischen Implantatversorgung mit formadaptiven Elementen, insbesondere Kunststoffimplantaten im Allgemeinen.

Medizinische Implantate werden in einer Vielzahl von Anwendungen eingesetzt. In manchen Anwendungen ist es erforderlich, dass sich das Implantat in einem bestimmten Körper-Hohlbereich besonders gut an die Geometrie des Hohlbereichs anpasst, z.B. bei Cochlea-Implantaten an die Form der Cochlea. So ist z.B. aus der WO 2015/188805 A1 für eine solche Anwendung ein selbstkrümmendes Implantat bekannt, bei dem ein schwellfähiges Polymer in einem Implantatgrundkörper vorhanden ist.

Flexible Kunststoffimplantate werden typischerweise im Kontakt mit Weichgewebestrukturen wie z.B. Hohlorganen oder Nervenstrukturen eingesetzt. Aufgrund ihrer Struktur und Materialkombination soll eine möglichst gute Anpassung an das Empfängergewebe erreicht und die bei der Implantation entstehenden Gewebeschäden minimiert werden. Gängige Weichgewebeimplantate bestehen daher meistens aus einem hochflexiblen Elastomer-Grundkörper, welcher im Falle eines aktiven Implantates zusätzlich mit Leiterstrukturen, wie z.B. Platindrähten, zur elektrischen Stimulation versehen ist. Ein bekannter Vertreter hierfür sind Cochlea-Implantatsysteme (CI), welche im Falle einer verlorenen Reiztransduktion im Innenohr (sensorineuraler Hörverlust) eingesetzt werden. Um das Gehör wiederherzustellen, wird ein Elektrodenträger in das Innenohr implantiert und die Nervenzellen elektrisch stimuliert. Für eine bessere Anpassung der Elektrodenträger an die Nervenstrukturen, werden vorgeformte Elektrodenträger eingesetzt, die die Schneckenform einer typischen Cochlea besitzen. Um die Elektrodenträger während der Implantation einführen zu können, muss der Elektrodenträger mit einem kleinen, eingelassenen Metalldraht (Stilett) gerade gehalten werden. Mit zunehmender Insertionstiefe wird der Metalldraht gezogen und die Elektrode bewegt sich in ihre vordefinierte Form zurück. Bisher nicht umgesetzte Forschungsansätze verfolgen die Verwendung von Shape Memory metallen und quellenden Polymeren [Patent US 8,145,326 B2], welche im letzteren Fall den Elektrodenträger durch eine Abstoßung auf der lateralen Seite in Richtung der Nervenzellen drücken sollen (perimodiolare Position). US 8 843 216 B2 offenbart einen Polymeraktor, der nach dem Prinzip einer elektromechanischen Zelle funktioniert, wobei der elektromechanische Prinzip insbesondere von einer Flüssigkeitszufuhr von außen unabhängig ist. US 2008/058914 A1 offenbart einen Aktor, der aus einem Hydrogel Segment besteht. Der Aktor steht jedoch in direktem Kontakt mit dem externen Flüssigkeitsmedium, ohne jegliches Flüssigkeitstransportmedium.

Der Erfindung liegt die Aufgabe zugrunde, solche formadaptiven medizinischen Implantate praxisgerechter und patientenschonender zu gestalten.

Diese Aufgabe wird bei dem eingangs genannten formadaptiven medizinischen Implantat gelöst durch eines, mehrere oder alle der folgenden Merkmale a), b), c), d):
a) das Implantat weist eine mit elektrischen Signalen beaufschlagbare elektrische Signalabgabeanordnung mit einer Anordnung aus elektrischen Leitern und/oder Elektroden auf, die zur Beaufschlagung der aufquellbaren chemischen Substanz des Aktors mit einem elektrischen und/oder elektromagnetischen Feld eingerichtet ist, wobei die aufquellbare chemische Substanz dazu eingerichtet ist, in Folge ihrer Beaufschlagung mit dem elektrischen und/oder elektromagnetischen Feld von der elektrischen Signalabgabeanordnung vom aufgequollenen Zustand in den nicht aufgequollenen Zustand überzugehen,
b) das Implantat weist ein flüssigkeitsdichtes Verschlussmittel aus einer flüssigkeitslöslichen chemischen Substanz auf, die das Flüssigkeitstransportmittel flüssigkeitsdicht verschließt,
c) das Implantat, insbesondere dessen Aktor, weist laterale Begrenzungsmittel auf, durch die die laterale Ausdehnung des Implantats und/oder des Aktors in Folge des Aufquellens der aufquellbaren chemischen Substanz begrenzt ist,
d) im Aktor, insbesondere in der aufquellbaren chemischen Substanz, ist ein ionisches Medikament zur gezielten Wirkstoffabgabe nach Implantation des Implantats im Körper vorhanden.

Die Erfindung hat den Vorteil, dass gemäß Merkmal a) das Explantationsverhalten des Implantats verbessert wird. Muss das Implantat im Falle einer Revisionsoperation wieder aus dem Körper, in den es implantiert wurde, entfernt werden, so kann dies derart erfolgen, dass durch Abgabe elektrischer Signale an die aufquellbare chemische Substanz, die sich im aufgequollenen Zustand befindet, diese wieder in den nicht aufgequollenen Zustand gebracht wird. Dementsprechend kann das Implantat im Wesentlichen ohne Gewebeschädigungen wieder entfernt werden.

Auf diese Weise kann das Implantat als elektrisch betätigbares oder elektrisch schaltbares medizinisches Implantat ausgebildet sein. Die zweite Implantat-Geometrie kann insbesondere eine gekrümmte geometrische Form sein. Durch das Abschwellen der aufquellbaren chemischen Substanz kann das Implantat insbesondere wieder die erste Implantat-Geometrie oder eine andere, von der zweiten Implantat-Geometrie abweichende geometrische Form einnehmen.

Die Flüssigkeit, durch die die aufquellbare chemische Substanz aufquillt, kann z.B. Wasser oder eine wässrige Substanz sein, insbesondere wässrige Substanzen im menschlichen Körper. Im Falle eines Cochlea-Implantats kann die Flüssigkeit eine wässrige intracochleare Flüssigkeit sein, die sich in der Cochlea befindet.

Die Erfindung hat den Vorteil, dass gemäß Merkmal b) die Flüssigkeitszufuhr zu der aufquellbaren chemischen Substanz in definierter Weise erfolgen kann. Das flüssigkeitsdichte Verschlussmittel kann z.B. in Form kleiner Körner randnah in einem Implantatgrundkörper eingebracht sein und sich bei Kontakt mit der Flüssigkeit auflösen. Die dabei freiwerdenden Stellen bilden bei entsprechend kleiner Korngröße der sich auflösenden flüssigkeitslöslichen chemischen Substanz eine poröse Membranstruktur am Implantatgrundkörper aus, die die gewünschten Trenneigenschaften zwischen der Flüssigkeit außerhalb des Implantats und dem mit der aufquellbaren chemischen Substanz gefüllten Aktor erfüllt. Die aufquellbare chemische Substanz kann somit den Aktor nicht verlassen, Flüssigkeit kann aber von außen durch die Membranstruktur ins Innere des Aktors gelangen und somit das Aufquellen der aufquellbaren chemischen Substanz bewirken.

Die Erfindung hat den Vorteil, dass gemäß Merkmal c) der Anpressdruck des Implantats an eine Hohlstruktur im Körper begrenzt werden kann, z.B. der Anpressdruck in der Cochlea. Auf diese Weise können durch das Aufquellen der aufquellbaren chemischen Substanz unerwünscht hohe Anpressdrücke und dementsprechende damit verbundene Risiken vermieden werden. Durch die lateralen Begrenzungsmittel wird die laterale Ausdehnung, mit anderen Worten, die Ausdehnung in radialer Richtung, begrenzt, z.B. auf einen vordefinierten maximalen Umfang des Implantats oder in flexibler Weise auf eine maximale Anpresskraft.

Die Erfindung hat den Vorteil, dass gemäß Merkmal d) das Implantat zugleich genutzt werden kann, um gezielt Wirkstoffe in den mit dem Implantat versorgten Körper abzugeben. Unter dem Einfluss eines elektrischen Feldes kann das Medikament beispielsweise in gezielten Dosen in die umgebende Flüssigkeit abgegeben werden.

Gemäß einer vorteilhaften Weiterbildung der Erfindung ist vorgesehen, dass die aufquellbare chemische Substanz ein polyelektrolytisches Hydrogel ist oder zumindest überwiegend aufweist. Auf diese Weise kann eine biokompatible aufquellbare chemische Substanz bereitgestellt werden. Das polyelektrolytische Hydrogel ist zudem reversibel vom aufgequollenen Zustand auch wieder in einen nicht aufgequollenen Zustand überführbar, und zwar durch Anlegen eines elektrischen Felds an dem polyelektrolytischen Hydrogel.

Gemäß einer vorteilhaften Weiterbildung der Erfindung ist vorgesehen, dass das Flüssigkeitstransportmittel eine membranartige Struktur und/oder Mikroporen der Außenhaut des Aktors aufweisen. Auf diese Weise ist die Außenhaut des Aktors durchlässig für die Flüssigkeit, die von außen in das Innere des Aktors dringen soll, um die aufquellbare chemische Substanz aufquellen zu lassen. Andererseits kann die aufquellbare chemische Substanz nicht aus dem Aktor austreten.

Gemäß einer vorteilhaften Weiterbildung der Erfindung ist vorgesehen, dass die flüssigkeitslösliche chemische Substanz des flüssigkeitsdichten Verschlussmittels Polyvinylpyrrolidon ist oder zumindest überwiegend aufweist. Auf diese Weise wird eine biokompatible flüssigkeitslösliche chemische Substanz als flüssigkeitsdichtes Verschlussmittel bereitgestellt. Entsprechende Polyvinylpyrrolidon-Zubereitungen sind beispielsweise unter dem Handelsnamen Luvitec der Firma BASF erhältlich.

Gemäß einer vorteilhaften Weiterbildung der Erfindung ist vorgesehen, dass das Implantat, insbesondere dessen Aktor, eine in die Außenhaut integrierte oder daran befestigte Faserverstärkung aufweist. Durch eine solche Faserverstärkung kann der Aktor robuster gestaltet werden. Zudem kann durch die Faserverstärkung das laterale Begrenzungsmittel realisiert werden. Die Faserverstärkung kann insbesondere an dem Aktor oberflächennah angeordnet sein oder in das Material des Aktors, d.h. dessen Hülle, integriert sein. Die Fasern können beispielsweise als Fäden oder Drähte ausgebildet sein.

Gemäß einer vorteilhaften Weiterbildung der Erfindung ist vorgesehen, dass die Faserverstärkung als unidirektionale Faserverstärkung ausgebildet ist, insbesondere als mit ihrer Faserrichtung in Querrichtung des Aktors verlaufende unidirektionale Faserverstärkung. Durch eine solche Faserrichtung und Art der Faserverstärkung kann insbesondere eine gewünschte longitudinale Ausdehnung des Aktors bei begrenzter lateraler Ausdehnung realisiert werden.

Gemäß einer vorteilhaften Weiterbildung der Erfindung ist vorgesehen, dass die elektrische Signalabgabeanordnung wenigstens zum Teil durch elektrisch leitende Fasern der Faserverstärkung gebildet ist. Dies hat den Vorteil, dass die Integration der elektrischen Signalabgabeanordnung in dem Implantat vereinfacht wird, bzw. keine zusätzlichen Elemente außer den Fasern der Faserverstärkung notwendig sind, um die elektrische Signalabgabeanordnung zu realisieren. Die elektrisch leitenden Fasern der Faserverstärkung können z.B. in Form von Kohlefasern ausgebildet sein.

Gemäß einer vorteilhaften Weiterbildung der Erfindung ist vorgesehen, dass die Fasern der Faserverstärkung eine gekettelte Struktur aufweisen. Hierdurch wird eine Struktur geschaffen, durch die die laterale Begrenzung der Ausdehnung des Aktors realisiert wird, ohne die gewünschte Dehnung in longitudinaler Richtung (axiale Richtung) zu unterdrücken.

Gemäß einer vorteilhaften Weiterbildung der Erfindung ist vorgesehen, dass das Implantat als Cochlea-Implantat ausgebildet ist, das zusätzlich zu der elektrischen Signalabgabeanordnung einen Elektrodenträger aufweist, an dem Stimulationselektroden zur Stimulation der Cochlea angeordnet sind. Auf diese Weise kann ein besonders patientenschonendes und hoch funktionelles Cochlea-Implantat bereitgestellt werden. In vorteilhafter Weise können die Stimulationselektroden und/oder deren elektrische Zuleitungen zugleich die elektrische Signalabgabeanordnung oder zumindest einen Teil davon bilden.

Die eingangs genannte Aufgabe wird ferner gelöst durch die Verwendung einer elektrischen Signalquelle zur Beaufschlagung der elektrischen Signalabgabeanordnung eines medizinischen Implantats der zuvor erläuterten Art mit elektrischen Signalen zur Explantierung des Implantats aus einem im Körper implantierten Zustand. Auch hierdurch können die zuvor erläuterten Vorteile realisiert werden. Die elektrische Signalquelle kann eine elektrische Signalquelle jeder Art sein, beispielsweise eine Gleichstromquelle, eine Gleichspannungsquelle oder eine Signalquelle, die ein elektrisches Wechselsignal abgibt.

Mit dem erfindungsgemäßen Implantat kann z.B. folgendes Verfahren realisiert werden.

Verfahren zur Explantierung eines medizinischen Implantats der zuvor erläuterten Art aus einem im Körper implantierten Zustand, mit folgenden Schritten:
a) Anschließen der elektrischen Signalanordnung des Implantats an eine elektrische Signalquelle,
b) Beaufschlagen der chemischen Substanz im Aktor über die elektrische Signalanordnung mit elektrischen Signalen während einer Einwirkzeit,
c) Entfernen des Implantats nach der Einwirkzeit aus dem Körper.

Auch hierdurch können die zuvor erläuterten Vorteile realisiert werden.

Die Erfindung wird nachfolgend anhand von Ausführungsbeispielen unter Verwendung von Zeichnungen näher erläutert.

Es zeigen
- Fig. 1 -: eine erste Ausführungsform der Erfindung und
- Fig. 2 -: eine zweite Ausführungsform der Erfindung.

In den Figuren werden gleiche Bezugszeichen für einander entsprechende Elemente verwendet.

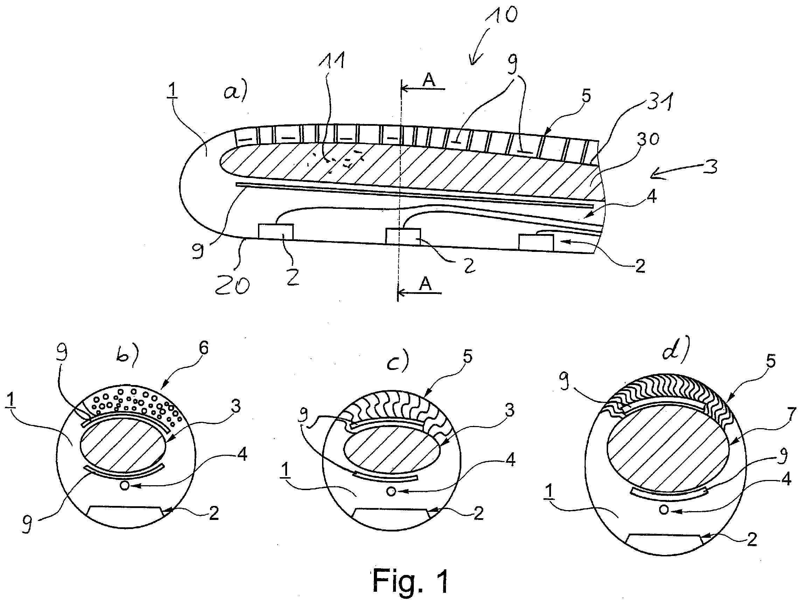

Die Figur 1 zeigt in der Abbildung a) ein formadaptives medizinisches Implantat in Form eines Cochlea-Implantats in seitlicher Schnittdarstellung. Die Abbildungen b) bis d) zeigen entsprechend der Schnittebene A- A das Implantat in verschiedenen Aufquellstadien der aufquellbaren chemischen Substanz.

Das Implantat 10 weist einen Grundkörper 1 auf, der z.B. aus Silikon gebildet sein kann, ferner Kontakte 2, elektrische Verbindungsleitungen 4, einen Aktor 3, gefüllt mit aufquellbarer chemischer Substanz 30, die in einer flexiblen Hülle 31 des Aktors 3 angeordnet ist. Die Kontakte 2 können die Elektroden bilden, z.B. Cochlea-Stimulationselektroden. Als Flüssigkeitstransportmittel 5 sind poröse Kanäle in der äußeren Struktur des Grundkörpers 1 vorhanden. Die Kontakte 2 und die Verbindungsleitungen 4 können z.B. aus inertem Material gebildet sein, z.B. aus Platin.

Die Abbildung a) zeigt einen Querschnitt durch das Implantat 10 im Herstellungszustand, d.h. das Implantat wurde noch nicht in einen Körper implantiert. Der mit der aufquellbaren chemischen Substanz 30 gefüllte Aktor 3 ist von der Umgebung durch einen durch den Grundkörper 1 gebildeten Silikonmantel abgeschlossen. Die porösen Kanäle 5 sind mit einem darin dispergierten wasserlöslichen Polymer 6, das als flüssigkeitsdichtes Verschlussmittel dient, abgeschlossen. Wie die Abbildung b) zeigt, löst sich das wasserlösliche Polymer 6 bei Kontakt mit einer wässrigen Lösung auf und hinterlässt die porösen Kanäle 5, somit eine feine poröse Struktur. Die poröse Struktur dient als permeabler Anschluss, der eine Wasserzufuhr von außen in den Aktor 3 ermöglicht, dabei aber ein Austreten des Hydrogels 30 aus dem Aktor 3 verhindert. Durch den Wasserzufluss schwillt das Hydrogel 30 und damit der gesamte Aktor 3 an, wie durch das Bezugszeichen 7 in der Abbildung d) dargestellt ist. Hierdurch wird eine Selbstkrümmung des Implantats und infolgedessen eine adaptive Konturanpassung des Implantats an einen Hohlraum in einem Körper, in dem das Implantat eingesetzt wurde, erzeugt.

Für die Verwendung der Erfindung in einem Elektrodenträger für Cochlea-Implantat-Systeme müsste der Elektrodenträger neu aufgebaut werden. Das bisherige Design eines zum Teil konischen Silikonzylinders mit darin eingelassenen Platinkontakten und Platindrähten müsste zusätzlich um den schwellenden Hydrogelaktor 3 und der selektiven permeablen Membran erweitert werden. Hierzu könnte auf den Elektrodenstrukturen ein Silikonkautschuk mit einem darin eingebrachten polyelektrolytitischen Hydrogel 30 appliziert und teilvulkanisiert werden. Darauf würde im nächsten Schritt ein Gemisch aus einem wasserlöslichen Pulver und einem Silikonkautschuk aufgebracht und vollständig vernetzt werden. Als polyelektrolytisches Hydrogel würde sich aufgrund seiner nachgewiesenen Biokompatibilität und Schwellfähigkeit ein Polyacrylamid anbieten, das mit ionischen Gruppen ergänzt ist. Das Polyacrylamid kann z.B. mit ionischen Gruppen in Form von Acrylsäuregruppen ergänzt werden. Für die poröse Membran würde z.B. Polyvinylpyrrolidon verwendet werden.

Nach der Implantation des Elektrodenträgers würde sich das Polyvinylpyrrolidon in der chlorhaltigen Innenohrflüssigkeit (Perilymphe) auflösen und eine definierte Porenstruktur 5 hinterlassen. Idealerweise könnte das innenliegende Hydrogel 30, aufgrund seiner vernetzten makroskopischen Struktur, die Poren nicht durchdringen, Wasser aber von der Innenohrflüssigkeit in das Hydrogel 30 strömen. Mit zunehmender Wasseraufnahme des Hydrogels 30 würde sich der Elektrodenträger an die Zielstrukturen anschmiegen und so den Kontakt zwischen den anvisierten Nervenzellen und den Elektrodenkontakten reduzieren.

Um im Falle einer nötigen Explantation die Schwellung des Hydrogels 30 zu reduzieren, könnten mit einer integrierten elektrischen Signalabgabeanordnung 9, ggf. mit den Elektrodenkontakten 2, ein elektrisches Feld über die Länge des Elektrodenträgers aufgebracht werden. Entsprechend lässt sich bei der Verwendung einer aufquellbaren chemischen Substanz in Form eines ionischen Polyacrylamides der Kollaps des Hydrogels mit einer Feldstärke von etwa 0,41 V/cm bis 1,66 V/cm innerhalb eines Tages realisieren.

Die aufquellbare chemische Substanz muss dabei nicht vollständig reversibel in den nicht aufgequollenen Zustand zurückführbar sein, ein teilweises Abquellen ist in vielen Anwendungen bereits ausreichend.

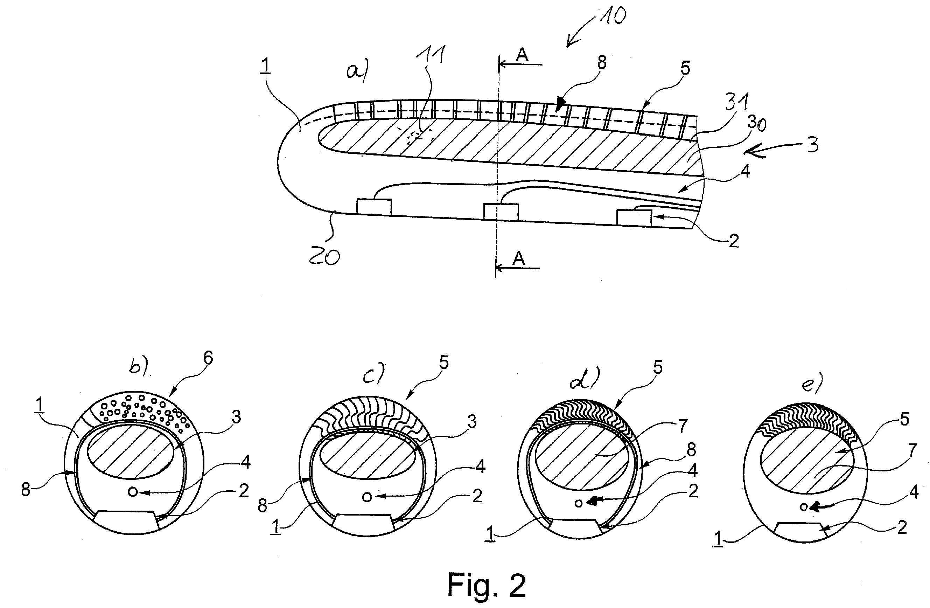

Die Figur 2 zeigt eine Ausführungsform der Erfindung, bei der das Implantat 10 zusätzlich noch laterale Begrenzungsmittel 8 aufweist, z.B. in Form von oberflächennahen unidirektionalen Verstärkungsfasern. Die Abbildung a) zeigt, ähnlich wie bei Figur 1, einen Längsschnitt durch das Implantat 10. Die Abbildungen b) und c) zeigen die gleichen Zustände wie die Abbildungen b) und c) der Figur 1.

In der Ausführungsform gemäß Figur 2 weist das Implantat keine gesonderte elektrische Signalabgabeanordnung 9 auf. Die elektrische Signalabgabeanordnung ist hier durch elektrisch leitende Fasern in Form der Verstärkungsfasern 8 gebildet.

Die Abbildung d) zeigt den Beginn des Anschwellvorgangs der aufquellbaren chemischen Substanz, ähnlich der Abbildung d) der Figur 1. Durch die Abbildung e) wird verdeutlicht, dass durch die Verstärkungsfasern 8, die oberflächennah im Grundkörper 1 an dessen Außenoberfläche angeordnet sind, die laterale Aufquellung des Implantats 10 begrenzt ist und gegenüber der ersten Ausführungsform deutlich reduziert ist.

Die Verstärkungsfasern 8 können als Kohlenstofffasern ausgebildet sein, z.B. mit einem Durchmesser in der Größenordnung von 7 Mikrometern. Hierdurch lassen sich Biegeradien im mittleren zweistelligen Mikrometerbereich ohne weiteres realisieren.

Durch die oberflächennahen Verstärkungsfasern 8 wird die gewünschte Dehnung in axialer Richtung im Wesentlichen nicht unterdrückt. Dementsprechend werden beispielsweise unidirektionale Fasern (Kette) ohne Querfaden (Schuss) im Grundkörper eingebracht, die zwar Kräfte in lateraler Richtung, aber nicht in axialer Richtung aufnehmen können.

Ein zweiter Teil der elektrischen Signalabgabeanordnung kann in beiden Ausführungsformen durch die Kontakte 2, ggf. in Verbindung mit den Verbindungsleitungen 4, gebildet sein.

Bei einem Implantat mit einem Durchmesser von z.B. 0,8 mm kann die erforderliche Feldstärke zum Abschwellen des Hydrogels schon mit einer Spannung von 0,066 Volt erreicht werden.

## Patentansprüche

1. Formadaptives medizinisches Implantat (10) mit wenigstens einem Aktor (3), durch den das Implantat (10) von einer ersten Implantat-Geometrie in eine zweite Implantat-Geometrie verstellt werden kann, wobei das Implantat (10) in der zweiten Implantat-Geometrie eine andere geometrische Form aufweist als in der ersten Implantat-Geometrie, wobei der Aktor (3) eine aufquellbare chemische Substanz (30) aufweist, die in Folge von Flüssigkeitszufuhr aufquillt, und wobei das Implantat wenigstens ein Flüssigkeitstransportmittel (5) aufweist, das zur Zuführung von außerhalb des Implantats vorhandener Flüssigkeit zu der aufquellbaren chemischen Substanz (30) eingerichtet ist, **gekennzeichnet durch** eines, mehrere oder alle der folgenden Merkmale a), b), c), d):
a) das Implantat (10) weist eine mit elektrischen Signalen beaufschlagbare elektrische Signalabgabeanordnung (9) mit einer Anordnung aus elektrischen Leitern und/oder Elektroden auf, die zur Beaufschlagung der aufquellbaren chemischen Substanz (30) des Aktors (3) mit einem elektrischen und/oder elektromagnetischen Feld eingerichtet ist, wobei die aufquellbare chemische Substanz (30) dazu eingerichtet ist, in Folge ihrer Beaufschlagung mit dem elektrischen und/oder elektromagnetischen Feld von der elektrischen Signalabgabeanordnung (9) vom aufgequollenen Zustand in den nicht aufgequollenen Zustand überzugehen,
b) das Implantat (10) weist ein flüssigkeitsdichtes Verschlussmittel (6) aus einer flüssigkeitslöslichen chemischen Substanz auf, die das Flüssig-keitstransportmittel flüssigkeitsdicht (5) verschließt,
c) das Implantat (10), insbesondere dessen Aktor (3), weist laterale Be-grenzungsmittel (8) auf, **durch** die die laterale Ausdehnung des Implantats (10) und/oder des Aktors (3) in Folge des Aufquellens der aufquell-baren chemischen Substanz (30) begrenzt ist,
d) im Aktor (3), insbesondere in der aufquellbaren chemischen Substanz (10), ist ein ionisches Medikament (11) zur gezielten Wirkstoffabgabe nach Implantation des Implantats (10) im Körper vorhanden.

2. Implantat nach dem vorhergehenden Anspruch, **dadurch gekennzeichnet, dass** die aufquellbare chemische Substanz (30) ein polyelektrolytisches Hydrogel ist oder zumindest überwiegend aufweist.

3. Implantat nach einem der vorhergehenden Ansprüche, **dadurch gekennzeichnet, dass** das Flüssigkeitstransportmittel (5) eine membranartige Struktur und/oder Mikroporen der Außenhaut (31) des Aktors (3) aufweisen.

4. Implantat nach einem der vorhergehenden Ansprüche, **dadurch gekennzeichnet, dass** die flüssigkeitslösliche chemische Substanz des flüssigkeitsdichten Verschlussmittels (6) Polyvinylpyrrolidon ist oder zumindest überwiegend aufweist.

5. Implantat nach einem der vorhergehenden Ansprüche, **dadurch gekennzeichnet, dass** das Implantat (10), insbesondere dessen Aktor (3), eine in die Außenhaut (31) integrierte oder daran befestigte Faserverstärkung (8) auf-weist.

6. Implantat nach dem vorhergehenden Anspruch, **dadurch gekennzeichnet, dass** die Faserverstärkung (8) als unidirektionale Faserverstärkung ausgebildet ist, insbesondere als mit ihrer Faserrichtung in Querrichtung des Aktors (3) verlaufende unidirektionale Faserverstärkung.

7. Implantat nach einem der Ansprüche 5 bis 6, **dadurch gekennzeichnet, dass** die elektrische Signalabgabeanordnung (9) wenigstens zum Teil durch elektrisch leitende Fasern der Faserverstärkung (8) gebildet ist.

8. Implantat nach einem der Ansprüche 5 bis 7, **dadurch gekennzeichnet, dass** die Fasern der Faserverstärkung (8) eine gekettelte Struktur aufweisen.

9. Implantat nach einem der vorhergehenden Ansprüche, dadurch gekenn-zeichnet, dass das Implantat (10) als Cochlea-Implantat ausgebildet ist, das zusätzlich zu der elektrischen Signalabgabeanordnung (9) einen Elektrodenträger (20) aufweist, an dem Stimulationselektroden (2) zur Stimulation der Cochlea angeordnet sind.

## Claims

1. A shape-adaptive medical implant (10) comprising at least one actuator (3) by means of which the implant (10) can be shifted from a first implant geometry to a second implant geometry, the implant (10) having a different geometric shape in the second implant geometry than in the first implant geometry, the actuator (3) having a swellable chemical substance (30) which swells as a consequence of supply of liquid, and the implant having at least one liquid-transport means (5) which is configured to supply liquid present outside the implant to the swellable chemical substance (30), **characterized by** one of, a plurality of or all of the following features a), b), c), d):
a) the implant (10) has an electrical signal-delivery arrangement (9) suppliable with electrical signals and comprising an arrangement of electrical conductors and/or electrodes, which is configured to supply the swellable chemical substance (30) of the actuator (3) with an electric and/or electromagnetic field, the swellable chemical substance (30) being configured to convert from the swollen state into the nonswollen state as a consequence of its supply with the electric and/or electromagnetic field by the electrical signal-delivery arrangement (9),
b) the implant (10) has a liquid-tight sealing means (6) composed of a liquid-soluble chemical substance which seals the liquid-transport means in a liquid-tight manner (5),
c) the implant (10), more particularly its actuator (3), has lateral limiting means (8), by means of which the lateral expansion of the implant (10) and/or of the actuator (3) as a consequence of the swelling of the swellable chemical substance (30) is limited,
d) the actuator (3), more particularly the swellable chemical substance (10), contains an ionic medicament (11) for the specific delivery of active ingredient after implantation of the implant (10) in the body.

2. The implant as claimed in the preceding claim, **characterized in that** the swellable chemical substance (30) is, or at least predominantly comprises, a polyelectrolytic hydrogel.

3. The implant as claimed in either of the preceding claims, **characterized in that** the liquid-transport means (5) has a membrane-type structure and/or micropores of the outer skin (31) of the actuator (3).

4. The implant as claimed in any of the preceding claims, **characterized in that** the liquid-soluble chemical substance of the liquid-tight sealing means (6) is, or at least predominantly comprises, polyvinylpyrrolidone.

5. The implant as claimed in any of the preceding claims, **characterized in that** the implant (10), more particularly its actuator (3), has a fiber reinforcement (8) which is integrated into the outer skin (31) or is attached thereto.

6. The implant as claimed in the preceding claim, **characterized in that** the fiber reinforcement (8) is in the form of a unidirectional fiber reinforcement, more particularly in the form of a unidirectional fiber reinforcement running with its fiber direction in the transverse direction of the actuator (3).

7. The implant as claimed in any of claims 5 to 6, **characterized in that** the electrical signal-delivery arrangement (9) is formed at least in part by electrically conductive fibers of the fiber reinforcement (8).

8. The implant as claimed in any of claims 5 to 7, **characterized in that** the fibers of the fiber reinforcement (8) have a linked structure.

9. The implant as claimed in any of the preceding claims, **characterized in that** the implant (10) is in the form of a cochlear implant which has, in addition to the electrical signal-delivery arrangement (9), an electrode support (20) on which stimulation electrodes (2) for the stimulation of the cochlea are arranged.

## Revendications

1. Implant médical (10) à forme adaptable, comprenant au moins un actionneur (3) par lequel l'implant (10) peut être déplacé d'une première géométrie d'implant à une deuxième géométrie d'implant, l'implant (10) présentant, dans la deuxième géométrie d'implant, une forme géométrique différente de la première géométrie d'implant, l'actionneur (3) possédant une substance chimique gonflable (30) qui gonfle en conséquence de l'acheminement d'un liquide, et l'implant possédant au moins un moyen de transport de liquide (5) qui est conçu pour l'acheminement d'un liquide présent à l'extérieur de l'implant vers la substance chimique gonflable (30), **caractérisé par** l'une, plusieurs ou la totalité des caractéristiques a), b), c), d) suivantes :
a) l'implant (10) possède un arrangement de délivrance de signal électrique (9) qui peut être soumis à des signaux électriques et qui comprend un arrangement de conducteurs électriques et/ou d'électrodes, qui sont conçus pour soumettre la substance chimique gonflable (30) de l'actionneur (3) à un champ électrique et/ou électromagnétique, la substance chimique gonflable (30) étant conçue pour, en conséquence d'avoir été soumise au champ électrique et/ou électromagnétique par l'arrangement de délivrance de signal électrique (9), transiter de l'état gonflé à l'état non gonflé,
b) l'implant (10) possède un moyen de fermeture (6) étanche aux liquides en une substance chimique soluble dans le liquide, qui ferme le moyen de transport de liquide (5) de manière étanche aux liquides,
c) l'implant (10), notamment son actionneur (3), possède des moyens de délimitation latéraux (8) par lesquels l'expansion latérale de l'implant (10) et/ou de l'actionneur (3) conséquente du gonflement de la substance chimique gonflable (30) est limitée,
d) un médicament ionique (11), destiné à une administration ciblée de substance active après l'implantation de l'implant (10), est présent dans l'actionneur (3), notamment dans la substance chimique gonflable (10).

2. Implant selon la revendication précédente, **caractérisé en ce que** la substance chimique gonflable (30) est, ou possède au moins principalement, un hydrogel polyélectrolytique.

3. Implant selon l'une des revendications précédentes, **caractérisé en ce que** le moyen de transport de liquide (5) possède une structure de type membrane et/ou des micropores de peau externe (31) de l'actionneur (3).

4. Implant selon l'une des revendications précédentes, **caractérisé en ce que** la substance chimique soluble dans le liquide du moyen de fermeture (6) étanche aux liquides est ou présente au moins principalement de la polyvinylpyrrolidone.

5. Implant selon l'une des revendications précédentes, **caractérisé en ce que** l'implant (10), notamment son actionneur (3), possède un renfort à fibres (8) intégré dans la peau externe (31) ou fixé à celle-ci.

6. Implant selon la revendication précédente, **caractérisé en ce que** le renfort à fibres (8) est réalisé sous la forme d'un renfort à fibres unidirectionnel, notamment sous la forme d'un renfort à fibres unidirectionnel dont la direction des fibres suit un tracé dans le sens transversal de l'actionneur (3).

7. Implant selon l'une des revendications 5 et 6, **caractérisé en ce que** l'arrangement de délivrance de signal électrique (9) est formé au moins en partie par des fibres électriquement conductrices du renfort à fibres (8).

8. Implant selon l'une des revendications 5 à 7, **caractérisé en ce que** les fibres du renfort à fibres (8) présentent une structure enchaînée.

9. Implant selon la revendication précédente, **caractérisé en ce que** l'implant (10) est réalisé sous la forme d'un implant cochléaire qui, en plus de l'arrangement de délivrance de signal électrique (9), possède un porte-électrodes (20) sur lequel sont disposées des électrodes de stimulation (2) destinées à la stimulation de la cochlée.
